(19) Europäisches Patentamt

European Patent Office

Office européen des brevets

(11) EP 1 736 712 A1

(12) EUROPEAN PATENT APPLICATION
published in accordance with Art. 158(3) EPC

(43) Date of publication:
27.12.2006 Bulletin 2006/52

(21) Application number: 05727427.6

(22) Date of filing: 28.03.2005

(51) Int Cl.:
*F24F 3/16* (2006.01)     *A61L 9/00* (2006.01)
*A61L 9/01* (2006.01)     *A61L 9/20* (2006.01)
*B01D 53/86* (2006.01)    *B01J 35/02* (2006.01)
*F24F 3/147* (2006.01)    *F24F 7/08* (2006.01)

(86) International application number:
PCT/JP2005/005727

(87) International publication number:
WO 2005/100867 (27.10.2005 Gazette 2005/43)

(84) Designated Contracting States:
AT BE BG CH CY CZ DE DK EE ES FI FR GB GR
HU IE IS IT LI LT LU MC NL PL PT RO SE SI SK TR

(30) Priority: 15.04.2004 JP 2004120843

(71) Applicant: DAIKIN INDUSTRIES, LTD.
Osaka-shi, Osaka 530-8323 (JP)

(72) Inventors:
• TAIRA, Shigeharu,
  Daikin Industries, Ltd.
  Kusatsu-shi, Shiga 525-8526 (JP)

• MATSUMOTO, Akihito,
  Daikin Industries, Ltd.
  Kusatsu-shi, Shiga 525-8526 (JP)
• KURODA, Tarou,
  Daikin Industries, Ltd.
  Kusatsu-shi, Shiga 525-8526 (JP)
• OKAMOTO, Yoshio,
  Daikin Industries, Ltd.
  Kusatsu-shi, Shiga 525-8526 (JP)

(74) Representative: Goddar, Heinz J. et al
FORRESTER & BOEHMERT
Pettenkoferstrasse 20-22
80336 München (DE)

(54) **HEAT EXCHANGE UNIT**

(57) It is an object of the present invention to provide a heat exchanging unit (100, 200) that can maintain, more effectively than a conventional heat exchanging unit, the cleanliness of a heat exchange element (12, 220). The heat exchanging unit (100, 200) comprises an indoor air exhaust passageway (8), an outdoor air supply passageway (9), a heat exchange element (12, 220), and a first air cleaning member (12b, 220b). The indoor air exhaust passageway (8) exhausts indoor air to an outdoor space as exhaust air (EA). The outdoor air supply passageway (9) supplies outdoor air to an indoor space as supply air (SA). The heat exchange element (12, 220) exchanges at least the sensible heat of the sensible heat and the latent heat between the exhaust air (EA) and the supply air (SA). In addition, the heat exchange element (12, 220) communicates with the indoor air exhaust passageway (8) and the outdoor air supply passageway (9). The first air cleaning member (12b, 220b) is provided to the supply airflow upstream side of the heat exchange element (12, 220). In addition, the first air cleaning member (12b, 220b) supports an apatite that has a photocatalytic function.

Fig. 4

## Description

### TECHNICAL FIELD

The present invention relates to a heat exchanging unit that efficiently recovers heat during ventilation.

### BACKGROUND ART

There is a technology (e.g., refer to Patent Document 1) that aims to prevent contamination of a heat exchange element of a total heat exchanging unit (due to, for example, dust, bacteria, viruses or the like suspended in the outdoor air) by disposing an air filter, whereon a photocatalyst is supported, on the supply airflow upstream side of the heat exchange element.

### PATENT DOCUMENT 1

Japanese Published Unexamined Patent Application No. H 10-311581

### DISCLOSURE OF THE INVENTION

### PROBLEMS SOLVED BY THE INVENTION

It is an object of the present invention to provide a heat exchanging unit that can prevent, more effectively than the conventional case, the contamination of a heat exchange element due to, for example, dust, bacteria, viruses or the like suspended in the outdoor air.

### MEANS FOR SOLVING THE PROBLEMS

A heat exchanging unit according to a first aspect of the present invention comprises an indoor air exhaust passageway, an outdoor air supply passageway, a heat exchange element and a first air cleaning member. Furthermore, the "heat exchanging unit" herein includes both a total heat exchanging unit and a sensible heat exchanging unit. The indoor air exhaust passageway exhausts indoor air to an outdoor space as exhaust air. The outdoor air supply passageway supplies outdoor air to an indoor space as supply air. The heat exchange element exchanges at least the sensible heat of the sensible heat and the latent heat between the exhaust air and the supply air. Furthermore, the heat exchange element may be a fixed type or a rotational type. In addition, "sensible heat" herein specifically means temperature. In addition, "latent heat" herein specifically means humidity. In addition, the heat exchange element communicates with the indoor air exhaust passageway and the outdoor air supply passageway. The first air cleaning member is provided on the supply airflow upstream side of the heat exchange element. In addition, the first air cleaning member supports apatite that has a photocatalytic function. Furthermore, the "air cleaning member" herein is, for example, an air filter, an electric precipitator or the like. In addition, the "apatite that has a photocatalytic function" herein is an apatite wherein, for example, some of the calcium atoms of calcium hydroxyapatite are substituted by titanium atoms by a technique such as ion exchange or the like. In addition, the apatite that has a photocatalytic function may be supported on an air cleaning member by mixing it into, for example, a resin, or may be supported on the air cleaning member by coating it thereon. In addition, the apatite that has a photocatalytic function may be activated by a light source, such as an ultraviolet light lamp or an LED, by outdoor or indoor light, or by a plasma discharger and the like.

Herein, the first air cleaning member is provided on the supply airflow upstream side of the heat exchange element. In addition, the first air cleaning member supports the apatite that has a photocatalytic function. The apatite that has a photocatalytic function exhibits a decomposition capacity that is greater than titanium dioxide with respect to, for example, bacteria, viruses and the like. Consequently, if the photocatalytic function of the apatite that has a photocatalytic function can be activated, then this heat exchanging unit can prevent, more effectively than a conventional heat exchanging unit, the contamination of the heat exchange element due to, for example, dust, bacteria, viruses or the like suspended in the outdoor air.

A heat exchanging unit according to a second aspect of the present invention is a heat exchanging unit according to a first aspect of the present invention, further comprising a second air cleaning member. The second air cleaning member is provided on the exhaust airflow upstream side of the heat exchange element. In addition, the second air cleaning member supports the apatite that has a photocatalytic function.

If the heat exchange element is a rotational type or a moisture permeable fixed type (i.e., a total heat exchanging type), then some of the exhaust air or the return air will mix with the supply air, and the bacteria, viruses and the like present in the exhaust air or the return air will unfortunately return back with the supply air.

However, the second air cleaning member herein is provided on the exhaust airflow upstream side of the heat exchange element. In addition, the second air cleaning member supports the apatite that has a photocatalytic function. Consequently, with this heat exchanging unit, if the photocatalytic function of the apatite that has a photocatalytic function can be activated, then the bacteria, viruses and the like present in the exhaust air or the return air will be decomposed, destroyed or deactivated by the time they arrive at the heat exchange element. Accordingly, this heat exchanging unit can prevent the return of bacteria, viruses, and the like present in the exhaust air or the return air back with the supply air, regardless of whether the heat exchange element is a rotational type or a moisture permeable fixed type. In addition, the heat exchanging unit can also effectively prevent the contamination of the heat exchange element due to, for example, dust, bacteria or viruses suspended in the indoor air.

A heat exchanging unit according to a third aspect of the present invention is a heat exchanging unit according to a first aspect or a second aspect of the present invention, further comprising a photocatalytic function activating part. The photocatalytic function activating part activates the photocatalytic function of the apatite. Furthermore, the "photocatalytic function activating part" herein is, for example, an ultraviolet light lamp, an LED, a plasma discharger (such as a glow discharger, a barrier discharger, or a streamer discharger) or the like.

Here, the photocatalytic function activating part activates the photocatalytic function of the apatite. Consequently, the heat exchanging unit can also be disposed in outdoor and indoor where light does not reach.

A heat exchanging unit according to a fourth aspect of the present invention is a heat exchanging unit according to a third aspect of the present invention, wherein the photocatalytic function activating part irradiates the apatite that has a photocatalytic function with light in a predetermined wavelength range. Furthermore, the "photocatalytic function activating part" herein is, for example, an ultraviolet light lamp, a purple LED or the like.

Here, the photocatalytic function activating part irradiates the apatite that has a photocatalytic function with light in a predetermined wavelength range. Consequently, it is possible to use, for example, an ultraviolet light lamp or an LED in order to activate the photocatalytic function of the apatite that has a photocatalytic function. Accordingly, the manufacturing cost of the heat exchanging unit can be held down.

A heat exchanging unit according to a fifth aspect of the present invention is a heat exchanging unit according to a third aspect of the present invention, wherein the photocatalytic function activating part is provided on the airflow upstream side of the air cleaning member. In addition, the photocatalytic function activating part supplies activators to the apatite that has a photocatalytic function. Furthermore, the "photocatalytic function activating part" herein is, for example, a glow discharger, a barrier discharger, a streamer discharger or the like. The "activators" herein are, for example, high speed electrons, ions, ozone, radicals such as hydroxy radicals, other excited molecules (such as excited oxygen molecules, excited nitrogen molecules, and excited water molecules) and the like.

Here, the photocatalytic function activating part is provided on the airflow upstream side of the air cleaning member. Furthermore, the photocatalytic function activating part supplies activators to the apatite that has a photocatalytic function. Consequently, with this heat exchanging unit, the high energy radicals can activate the photocatalytic function of the apatite that has a photocatalytic function. Accordingly, it is possible to accelerate the photocatalytic reaction speed of the apatite that has a photocatalytic function. As a result, the heat exchanging unit can prevent, more effectively than a conventional heat exchanging unit, the contamination of the heat exchange element due to, for example, dust, bacteria, viruses or the like suspended in the outdoor air. In addition, it is known that ozone is extremely effective against a bactericide and the like. Consequently, the heat exchanging unit can prevent, more effectively than a conventional heat exchanging unit, the contamination of the heat exchange element due to, for example, dust, bacteria, viruses or the like suspended in the outdoor air.

A heat exchanging unit according to a sixth aspect of the present invention is a heat exchanging unit according to a fifth aspect of the present invention, further comprising an air distribution part and an operation switching unit. Furthermore, the "air distribution part" herein is, for example, a fan. The air distribution part distributes the air. The operation switching unit switches the operation state of the air distribution part. Furthermore, the "operation switching unit" herein is, for example, a control device that switches the rotational speed of the impeller of a fan.

Here, the operation switching unit switches the operation state of the air distribution part. Consequently, a high concentration of activators is supplied to the air cleaning member if the photocatalytic function activating part is used in a state wherein, for example, during normal operation, dust, bacteria, viruses and the like are captured in advance by the air cleaning member without making use of the photocatalytic function activating part, operation is then switched to a cleaning operation mode after a fixed time, and the air volume of the fan is maximally suppressed. Accordingly, with this heat exchanging unit, the air cleaning member can be cleaned selectively and effectively.

## EFFECTS OF THE INVENTION

If the photocatalytic function of the apatite that has a photocatalytic function can be activated, then the heat exchanging unit of the first aspect of the invention can prevent, more effectively than a conventional heat exchanging unit, the contamination of the heat exchange element due to, for example, dust, bacteria, viruses or the like suspended in the outdoor air.

With the heat exchanging unit of the second aspect of the invention, if the photocatalytic function of the apatite that has a photocatalytic function can be activated, then the bacteria, viruses and the like present in the exhaust air or the return air will be decomposed, destroyed or deactivated by the time they arrive at the heat exchange element. Accordingly, this heat exchanging unit can prevent the return of bacteria, viruses, and the like present in the exhaust air or the return air back with the supply air, regardless of whether the heat exchange element is a rotational type or a moisture permeable fixed type. In addition, the heat exchanging unit can also effectively prevent the contamination of the heat exchange element due to, for example, dust, bacteria, viruses or the like suspended in the indoor air.

The heat exchanging unit of the third aspect of the invention can also be disposed in outdoor and indoor where light does not reach.

The manufacturing cost of the heat exchanging unit according to the fourth aspect of the invention can be held down.

The heat exchanging unit according to the fifth aspect of the invention can prevent, more effectively than a conventional heat exchanging unit, the contamination of the heat exchange element due to, for example, dust, bacteria, viruses or the like suspended in the outdoor air. In addition, it is known that ozone is extremely effective against a bactericide and the like. Consequently, the heat exchanging unit can prevent, more effectively than a conventional heat exchanging unit, the contamination of the heat exchange element due to, for example, dust, bacteria, viruses or the like suspended in the outdoor air.

With the heat exchanging unit according to the sixth aspect of the invention, the air cleaning member can be cleaned selectively and effectively.

## BRIEF DESCRIPTION OF THE DRAWINGS

FIG. 1 is an oblique view that shows the internal structure of a total heat exchanging unit according to the present invention.

FIG 2 is a top view that shows the internal structure of a total heat exchanging unit according to the present invention.

FIG. 3 is a side view that shows the internal structure of a total heat exchanging unit according to the present invention.

FIG. 4 is an exploded oblique view that shows the internal structure of the total heat exchanging unit according to the present invention.

FIG. 5 is an oblique view of a partition plate.

FIG. 6 is an oblique view of a partition plate.

FIG. 7 is an oblique view that shows the structure of a heat exchange element.

FIG. 8 is an oblique view that shows the internal structure of the heat exchanging unit according to a modified example (B).

FIG. 9 is an oblique view that shows the structure of the heat exchange element according to a modified example (B).

## EXPLANATION OF SYMBOLS

| 8 | Exhaust air passageway (indoor air exhaust passageway) |
|---|---|
| 9 | Supply air passageway (outdoor air supply passageway) |
| 10,11,210,211 | Fans (air distribution part) |
| 12, 220 | Heat exchange elements |
| 12b, 220b | Air filters (first air cleaning member, second air cleaning member) |
| 15 | Ultraviolet light lamp (photocatalytic function activating part) |
| 100, 200 | Total heat exchanging units (heat exchanging units) |
| EB | Electrical equipment box (operation switching unit) |
| EA | Exhaust air |
| SA | Supply air |

## BEST MODE FOR CARRYING OUT THE INVENTION

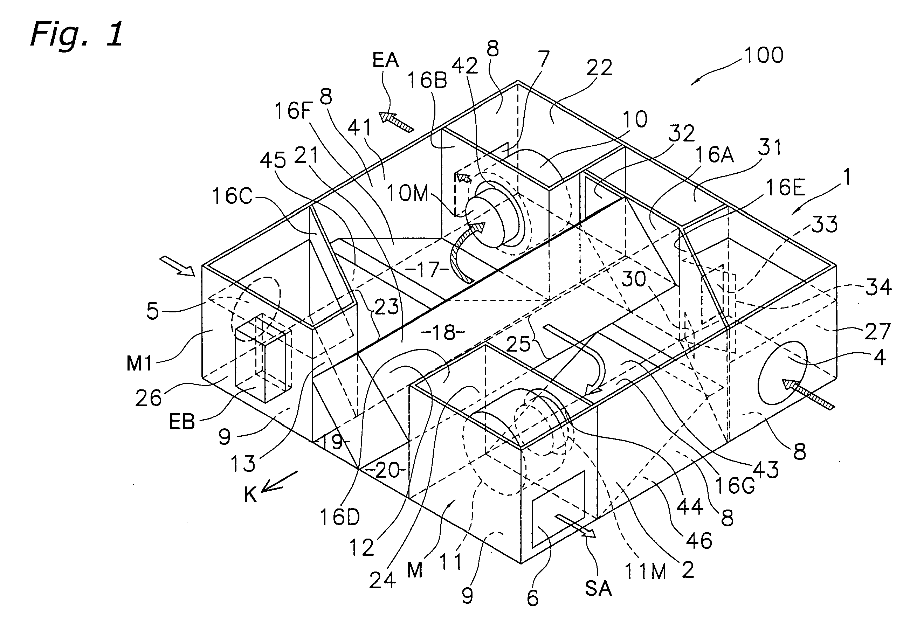

FIGS. 1, 2, 3, and 4 show an oblique view, a top view, a side view, and an exploded oblique view, respectively, of the internal structure of a total heat exchanging unit according to one embodiment of the present invention. Furthermore, as shown in FIG. 1, a total heat exchanging unit 100 is an apparatus for ventilating an indoor space while exchanging heat between supply air SA (solid line outline arrow) from an outdoor space and exhaust air EA (hatched outline arrow) from the indoor space via a heat exchange element 12.

**(CONSTITUTION OF THE TOTAL HEAT EXCHANGING UNIT)**

As shown in FIGS. 1, 2, 3, and 4, the present total heat exchanging unit 100 principally comprises a casing 1, a heat exchange element 12, air filters 12b, ultraviolet light lamps 15, fans 10, 11, a damper 34, and an electrical equipment box EB.

**(CONSTITUENT ELEMENTS OF THE TOTAL HEAT EXCHANGING UNIT)**

**(1) CASING**

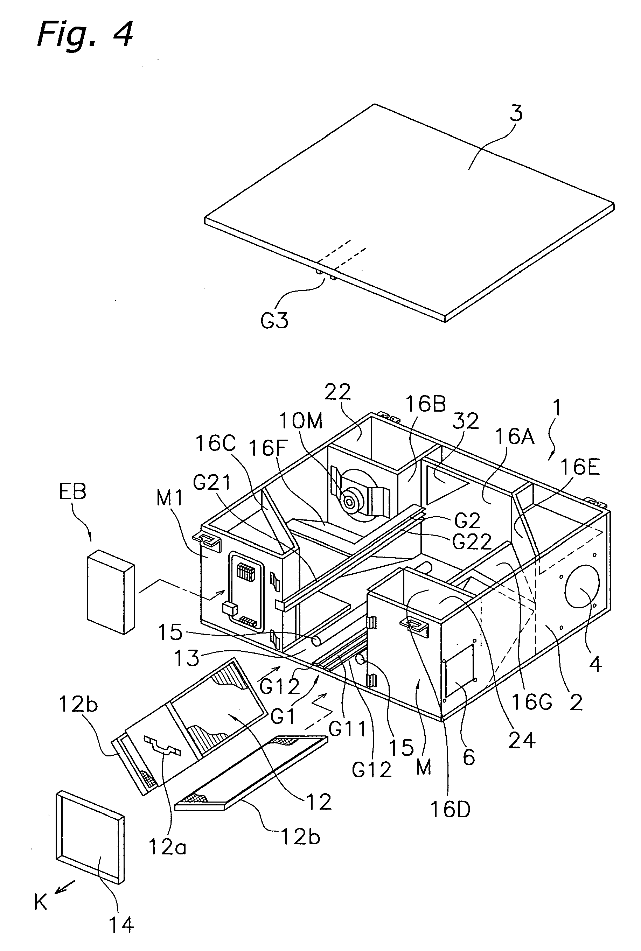

As shown in FIG. 1 and FIG. 4, the casing 1 comprises a casing body 2 and a cover body 3, which covers the upper surface of the casing body 2. Furthermore, a heat exchange element chamber 21, an exhaust air fan motor housing chamber 41, an exhaust air fan housing chamber 22, a supply air fan motor housing chamber 43, a supply air fan housing chamber 24, a supply air communicating chamber 45, an exhaust air communicating chamber 46, an outdoor side suction chamber 26, an indoor side suction chamber 27, and a bypass chamber 31 are provided in the casing 1. The following describes the details of each of the chambers discussed above.

**A. HEAT EXCHANGE ELEMENT CHAMBER**

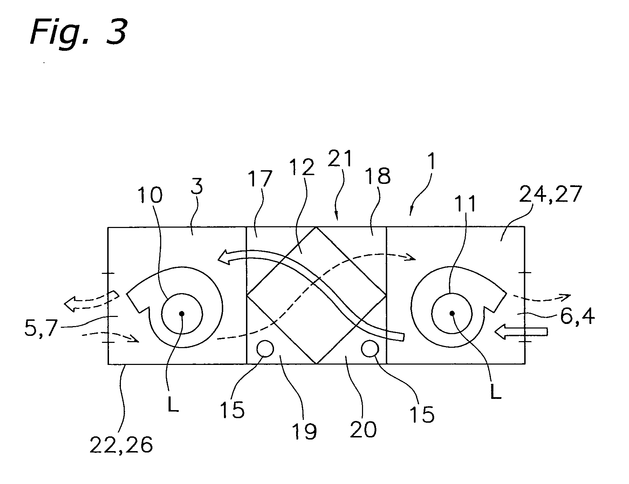

The heat exchange element chamber 21 is a rectangular parallelepipedic space, as shown in FIG. 1 and FIG. 3, and houses the heat exchange element 12. Furthermore, the heat exchange element chamber 21 is formed so that it is partitioned by, for example, a bottom plate of the casing body 2, partition plates 16A-16E (refer to FIG. 1, 5, and 6), and the cover body 3. In addition, guide parts G1, G2, G3 are affixed to the bottom plate of the casing body 2, the partition plates 16A-16E, and the cover body 3, respectively. The guide part G1, which is affixed to the bottom plate of the casing body 2, comprises a first guide part G11 and second guide parts G12. The first guide part G11 guides the ridgeline of the lower part of the heat exchange element 12 when the heat exchange element 12 is inserted or removed. Moreover, a pair of second guide parts G12 is disposed so that the first guide part G11 is interposed therebetween, and guides the end edges of the pair of air filters 12b. The guide part G2, which is affixed to the partition plates 16A-16E, comprises a first guide part G21 and a second guide part G22. The first guide part G21 guides a ridgeline of a side part of the heat exchange element 12 when the heat exchange element 12 is inserted or removed. Moreover, the second guide part G22 guides the end edges of the air filters 12b. The guide part G3, which is affixed to the cover body 3, guides a ridgeline of an upper part of the heat exchange element 12 when the heat exchange element 12 is inserted or removed.

Furthermore, housing the heat exchange element 12 in the heat exchange element chamber 21 creates four substantially triangular prism shaped spaces 17, 18, 19, 20 therein. Hereinbelow, the spaces indicated by reference symbols 17, 18, 19, and 20 in FIG. 1 and FIG. 3 are called a first space, a second space, a third space, and a fourth space, respectively.

**B. EXHAUST AIR FAN HOUSING CHAMBER**

The exhaust air fan housing chamber 22 houses the exhaust air fan 10, as shown in FIG. 1 and FIG. 4. In addition, as shown in FIG. 1, the exhaust air fan housing chamber 22 communicates with the exhaust air fan motor housing chamber 41 via an opening 42, which is formed in the partition plate 16B. In addition, as shown in FIG. 1, the exhaust air fan housing chamber 22 comprises an exhaust air outdoor side blow out port 7 on its side wall.

**C. EXHAUST AIR FAN MOTOR HOUSING CHAMBER**

The exhaust air fan motor housing chamber 41 houses an exhaust air fan motor 10M, as shown in FIG. 1 and FIG 4. In addition, the exhaust air fan motor housing chamber 41 communicates with the first space 17 via an opening 23, which is partitioned by the cover body 3 and one of the ridgelines of the heat exchange element 12, as shown in FIG. 1.

**D. SUPPLY AIR FAN HOUSING CHAMBER**

The supply air fan housing chamber 24 houses the supply air fan 11, as shown in FIG. 1 and FIG. 4. In addition, the supply air fan housing chamber 24 communicates with the supply air fan motor housing chamber 43 via an opening 44, which is formed in the partition plate 16D, as shown in FIG. 1. In addition, the supply air fan housing chamber 24 comprises a supply air indoor side blow out port 6 on its side wall, as shown in FIG. 1.

### E. SUPPLY AIR FAN MOTOR HOUSING CHAMBER

The supply air fan motor housing chamber 43 houses a supply air fan motor 11 M, as shown in FIG. 1 and FIG. 4. In addition, the supply air fan motor housing chamber 43 communicates with the second space 18 via an opening 25, which is partitioned by the cover body 3 and one of the ridgelines of the heat exchange element 12.

### F. OUTDOOR SIDE SUCTION CHAMBER

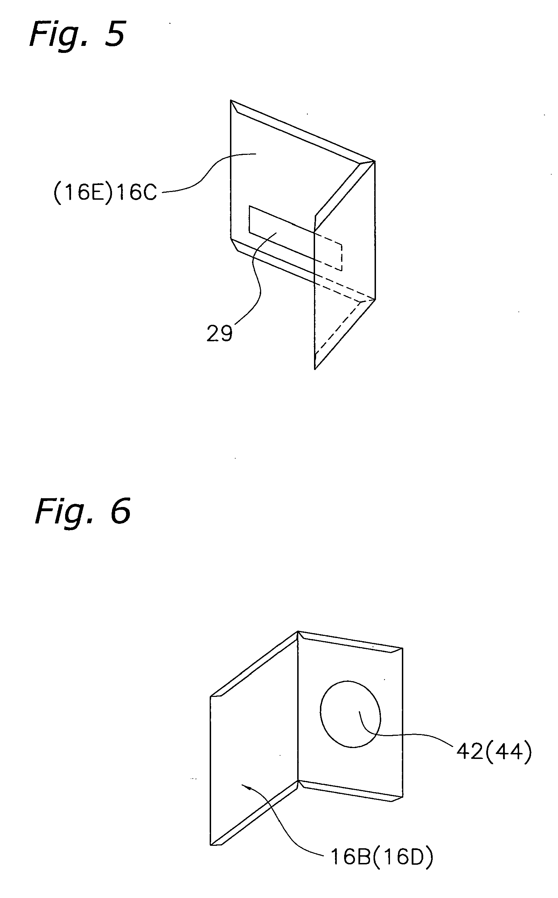

The outdoor side suction chamber 26 comprises a supply air outdoor side suction port 5 on its side wall, as shown in FIG. 1. In addition, the outdoor side suction chamber 26 communicates with the supply air communicating chamber 45 via an opening 29 of the partition plate 16C, as shown in FIG. 1 and FIG. 5.

### G. INDOOR SIDE SUCTION CHAMBER

The indoor side suction chamber 27 comprises an exhaust air indoor side suction port 4 on its side wall, as shown in FIG. 1. In addition, the indoor side suction chamber 27 communicates with the exhaust air communicating chamber 46 via an opening 30 of the partition plate 16E, as shown in FIG. 1.

### H. SUPPLY AIR COMMUNICATING CHAMBER

The supply air communicating chamber 45 is partitioned by the partition plate 16F, and is positioned below the exhaust air fan motor housing chamber 41. In addition, the supply air communicating chamber 45 communicates with the third space 19, as shown in FIG. 1.

### I. EXHAUST AIR COMMUNICATING CHAMBER

The exhaust air communicating chamber 46 is partitioned by the partition plate 16G, and is positioned below the supply air fan motor housing chamber 43. In addition, the exhaust air communicating chamber 46 communicates with the fourth space 20, as shown in FIG. 1.

### J. BYPASS CHAMBER

The bypass chamber 31 is positioned on the side opposite an extraction direction k of the heat exchange element chamber 21. Furthermore, the bypass chamber 31 communicates with the first space 17 via an opening 32. In addition, the bypass chamber 31 communicates with the indoor side suction chamber 27 via an opening 33. As a result, the exhaust air fan housing chamber 22 and the indoor side suction chamber 27 communicate via the exhaust air fan motor housing chamber 41, the first space 17, and the bypass chamber 31.

### (2) HEAT EXCHANGE ELEMENT

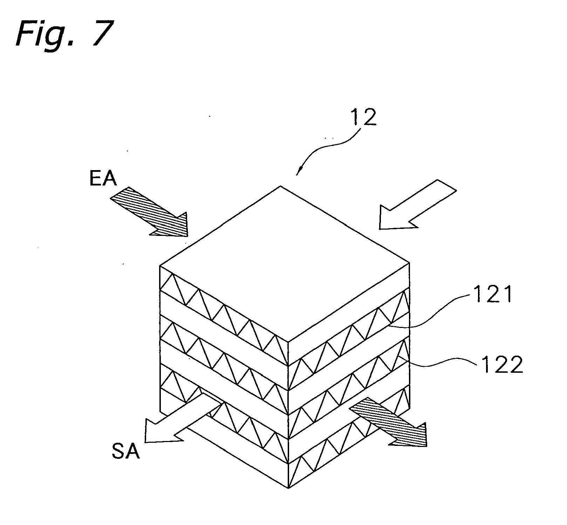

The heat exchange element 12 is formed substantially as a rectangular parallelepiped, as shown in FIG. 1 and FIG. 4, and is provided to the intersecting portion between an exhaust air passageway 8 and a supply air passageway 9. The heat exchange element 12 has a laminar structure, wherein special pleated kraft paper (hereinbelow, referred to as spacer paper) 122 and special flat sheet shaped kraft paper (hereinbelow, referred to as partitioning paper) 121 are stacked in alternating directions, as shown in FIG. 7. Because the heat exchange element 12 is so structured, passageways in the heat exchange element 12 for the exhaust air EA and the supply air SA are disposed so that they alternate by level. Furthermore, with the heat exchange element 12, the sensible heat and the latent heat of the supply air SA and the exhaust air EA are exchanged via the partitioning paper 121.

Furthermore, a handle 12a for removing the heat exchange element 12 is provided to an end surface of the heat exchange element 12, which can be inserted or removed in the longitudinal direction along its long side via an insertion/removal opening 13, which is open to a maintenance surface M of the casing 1, by removing a cover 14 as shown in FIG. 4.

### (3) AIR FILTER

As shown in FIG. 4, the air filters 12b are affixed to the heat exchange element 12 so that they cover the surfaces of the heat exchange element 12 that contact the third space 19 and the fourth space 20. Each of the air filters 12b is a nonwoven fabric that is made of polypropylene fibers. Each polypropylene fiber comprises a core part and an enveloping layer, and titanium apatite is supported on the enveloping layer. Furthermore, titanium apatite is an apatite wherein some of the calcium atoms of a calcium hydroxyapatite are substituted with titanium atoms using a technique such as ion exchange; titanium apatite exhibits excellent adsorption performance with respect to organic substances (particularly bacteria, viruses, and the like) and also exhibits properties of a photosemiconductor catalyst; in addition, if irradiated with light of a wavelength that has a high energy level (e.g., ultraviolet light and the like), then titanium apatite exhibits contaminant decomposition processing performance as a photosemiconductor catalyst superior to that of typical titanium dioxide of the anatase form.

## (4) ULTRAVIOLET LIGHT LAMPS

The ultraviolet light lamps 15 are provided to the third space 19 and the fourth space 20, respectively, and the supply of ultraviolet light thereby activates the photocatalytic function of the titanium apatite supported on the air filters 12b.

## (5) FAN

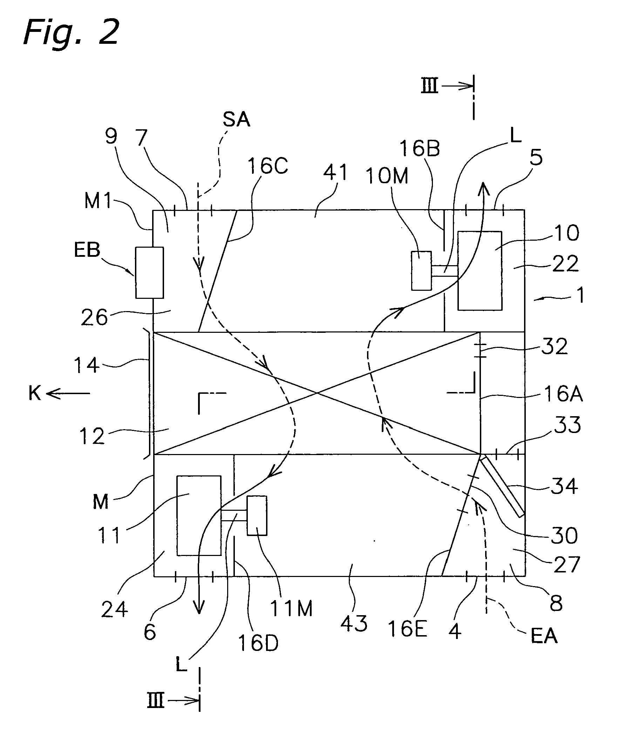

The exhaust air fan 10 and the supply air fan 11 are sirocco fans (rotors), as shown in FIG. 2 and FIG. 3, and are housed inside a curled fan casing (not shown) that is made of a resin foam (e.g., expanded polystyrene). Furthermore, a rotational axis line L of each of the fans 10, 11 is parallel to the extraction direction K of the heat exchange element 12.

## (6) DAMPER

The damper 34 is disposed inside the indoor side suction chamber 27. The damper 34 is pivoted by, for example, an electric motor (not shown) that opens either the opening 30 or the opening 33 and blocks the other.

## (7) ELECTRICAL EQUIPMENT BOX

The electrical equipment box EB is disposed in a portion M1, which opposes the exhaust air fan 10, of the maintenance surface M. The electrical equipment box EB houses electrical equipment, such as a control circuit board (not shown). Furthermore, the control circuit board is connected to and in communication with a wired remote control (not shown), and controls the operation of the fans 10, 11 and the damper 34 based on signals sent from the wired remote control.

## (PERFORMANCE OF TITANIUM APATITE ON BACTERIA AND VIRUSES)

Table 1 shows the percentage of viruses, bacteria, and toxins inactivated by titanium apatite.

**TABLE 1**

| Target Sample | | Inactivity Percentage | Testing Institution Certification Number |
|---|---|---|---|
| Influenza virus | | ≥ 99.99% | Japan Food Research Laboratories No. 203052102 |
| Antimicrobial | Escherichia coli (O157) | ≥ 99.99% | Japan Food Research Laboratories No. 203030567-001 |
| | Staphylococcus aureus | ≥ 99.99% | Japan Food Research Laboratories No. 203030567-001 |
| | Cladosporium | ≥ 99.99% | Japan Food Research Laboratories No. 203030567-001 |
| Toxin | Enterotoxin | ≥ 99.9% | Japan Food Research Laboratories No. 203050715-001 |

Furthermore, these inactivity percentages were measured at Japan Food Research Laboratories by the methods described below.

## 1. INACTIVITY PERCENTAGE OF INFLUENZA VIRUS

### (1) TEST OVERVIEW

An influenza virus suspension was dropped onto titanium apatite-coated filters (approximately 30 x 30 mm) and preserved at room temperature under dark (shielded from light) and light (black light irradiation with a distance of approximately 20 cm between the filter and the black light) conditions; after 24 hours, the virus infectivity titer was measured.

### (2) CALCULATION OF INACTIVITY PERCENTAGE

$$\text{Inactivity percentage} = 100 \times (1 - 10^{B}/10^{A})$$

A: Virus infectivity titer immediately after seeding
B: Virus infectivity titer of the filter after 24 hours of light irradiation

### (3) TEST METHOD

**A.** Test virus: Influenza virus type A (H1N1)

**B.** Cells: MDCK (NBL-2) cells, ATCC CCL-34 (Dainippon Pharmaceutical Co., Ltd.)

**C.** Culture medium

a) Cell growth medium: Eagle MEM (containing 0.06 mg/mL of Kanamycin) to which 10% newborn bovine serum was added.
b) Cell maintenance medium

The following tissue culture was used:

| | |
|---|---|
| Eagle MEM | 1,000 mL |
| NaHCO$_3$ (10%) | 24-44 mL |
| L-glutamine (30 g/L) | 9.8 mL |
| Vitamin solution for 100 $\times$ MEM | 30 mL |
| Albumin (10%) | 20 mL |
| Trypsin (5 mg/mL) | 2 mL |

**D.** Preparation of virus suspension

a) Cell culture
A monolayer culture of MDCK cells was incubated in a tissue culture flask using a cell growth medium.
b) Seeding of virus
After monolayer incubation, the cell growth medium was eliminated from the flask, and the culture was seeded with the test virus. Next, the cell maintenance medium was added and then incubated for 2 to 5 days at 37° C in a CO$_2$ incubator (CO$_2$ concentration of 5%).
c) Preparation of virus suspension

After incubation, an inverted phase contrast microscope was used to observe the cytomorphology, and a morphological change (cytopathic effect) was confirmed in over 80% of the cells. Next, the culture solution was centrifugally isolated (3,000 rpm for 10 min), and the obtained supernatant liquid was used as the virus suspension.

**E.** Preparation of sample

After the filter (approximately 30 x 30 mm) was sterilized with moist heat (121° C for 15 min), it was air dried for one hour, placed in a plastic Petri dish, irradiated with black light (black light blue, two FL20S BL-B 20W bulbs in parallel) for over 12 hours, and then used as a sample.

**F.** Test operation

0.2 mL of the virus suspension was dropped onto the sample. The samples were maintained at room temperature under light shielded and black light irradiation (distance of approximately 20 cm between the filter and the black light) conditions. In addition, polyethylene film was used as the control sample, and tested in the same manner.

**G.** Washing out the virus

After the above conditions were preserved for 24 hours, 2 mL of the cell maintenance medium was used to wash out the virus suspension in the test specimen.

**H.** Measurement of virus infectivity titer

The cell growth medium was used to incubate a monolayer culture of MDCK cells in a tissue culture microplate (96 wells), after which the cell growth medium was eliminated and the cell maintenance medium was added 0.1 mL at a time. Next, a washout liquid and 0.1 mL of a diluted solution thereof were used to seed 4 wells each, which were then incubated for 4 to 7 days at 37° C in a $CO_2$ incubator (5% $CO_2$ concentration). After incubation, an inverted phase contrast microscope was used to observe the presence of cytomorphological change (cytopathic effect), and the 50% tissue culture infectious dose ($TCID_{50}$) was computed using the Reed-Muench method and then used the result to calculate the virus infectivity titer per milliliter of the washout liquid.

## 2. INACTIVITY PERCENTAGES OF ESCHERICHIA COLI (O157), STAPHYLOCOCCUS AUREUS, AND CLADOSPORIUM

### (1) TEST OVERVIEW

The antimicrobial activity of filters were tested, referencing the test method of the Society of Industrial-Technology for Antimicrobial Articles, i.e., *Assay Antimicrobial Activity III for Antimicrobial Processed Products (2001 Edition) Light Irradiation Film Contact Method* (hereinafter, referred to as the "Light Irradiation Film Contact Method - SIAA 2001 Edition").
Furthermore, testing was performed as follows.
Bacterial suspensions of Escherichia coli, staphylococcus aureus, and Cladosporium were dropped onto samples, which were then sealed by covering them with a low density polyethylene film. These samples were preserved at room temperature (20°-25° C) under dark (shielded from light) and light (black light irradiation with a distance of approximately 20 cm between the filters and the black light) conditions; after 24 hours, the viable cell count was measured.

### (2) TEST METHOD

**A.** Test strain

Microbes:

Escherichia coli IFO 3972
Staphylococcus aureus subspecies aureus IFO 12732

Fungi:

Cladosporium cladosporioides IFO 6348

**B.** Test culture

NA medium: Nutrient agar (Eiken Chemical Co., Ltd.)
1/500 NB medium: Nutrient broth (Eiken Chemical Co., Ltd.) supplemented with 0.2% meat extract was diluted 500 times with a phosphate buffer to adjust the pH to 7.0 ±0.2.
SCDLP medium: SCDLP medium (Nippon Pharmaceutical Co., Ltd.)
SA medium: Standard agar (Eiken Kizai Co., Ltd.)
PDA medium: Potato dextrose agar medium (Eiken Kizai Co., Ltd.)

**C.** Preparation of bacterial suspensions

Microbes
Test strains were incubated beforehand in the NA medium at 35° C for 16 to 24 hours, the NA medium was reseeded with that culture, and the bacteria bodies, which were incubated at 35° C for 16 to 20 hours, were evenly dispersed in the 1/500 NB medium, and then prepared so that the bacteria count per milliliter was $2.5 \times 10^5$ to $1.0 \times 10^6$.
Fungi
After incubation in a PDA medium for 7 to 10 days at 25° C, the spores (conidias) were suspended in a 0.005% dioctyl sodium sulfosuccinate solution, filtered with gauze, and then prepared so that the spore count per milliliter was $2.5 \times 10^5$ to $1.0 \times 10^6$.

**D.** Preparation of samples

After each filter (approximately $50 \times 50$ mm) was sterilized with moist heat (121° C for 15 min), it was air dried for one hour, placed in a plastic Petri dish, irradiated for over 12 hours with black light (black light blue, two FL20S BL-B 20W bulbs in parallel), and then used as a sample.

**E.** Test operation

0.4 mL of each bacterial suspension was dropped onto a sample, which was then sealed by covering it with low density polyethylene film ($40 \times 40$ mm). The samples were preserved at room temperature (20°-25° C) under light shielded and black light irradiation (with a distance of approximately 20 cm between the filter and the black light) conditions. In addition, the polyethylene film was used as the control sample and tested in the same manner.

**F.** Measurement of viable cell count

After the above conditions were preserved for 24 hours, the surviving bacteria were washed out from each sample with the SCDLP medium and the viable cell count of the washout liquid was measured by the poured plate culture method, wherein the SA medium was used for the microbes (incubated for 2 days at 35° C) and the PDA medium was used for the fungi (incubated for 7 days at 25° C); thereafter, the viable cell count per sample was calculated. In addition, immediately after seeding, this measurement was performed on the control sample.

## 3. INACTIVITY PERCENTAGE OF THE ENTEROTOXIN

### (1) TEST OVERVIEW

The samples were seeded with staphylococcal enterotoxin A (hereinafter, abbreviated as "SET-A") and then preserved at room temperature (20°-25° C) under dark (shielded from light) and light (irradiated with ultraviolet light with a luminous flux of approximately 1 mW/cm$^2$) conditions; 24 hours later, the concentration of the SET-A was measured and the decomposition percentage was calculated.

### (2) TEST METHOD

**A.** Preparation of standard stock solution

A 5 μg/mL standard stock solution was prepared by dissolving a SET-A reference sample (Toxin Technology Inc.) in a 1% sodium chloride solution containing 0.5% bovine serum albumin.

**B.** Standard solution used for the calibration curve

Standard solutions of 0.2, 0.5, and 1 ng/mL were prepared by diluting the standard stock solution with the buffer solution included with the VIDAS staphylococcus enterotoxin (SET) test kit (bioMérieux, Inc.).

**C.** Preparation of samples

The filter was cut into 50 × 50 mm pieces, irradiated for 24 hours with a black light from a distance of approximately 1 cm, and then used as the samples.

**D.** Test operation

Each sample was placed in a plastic Petri dish and seeded with 0.4 mL of SET-A standard stock solution. The samples were preserved at room temperature (20°-25° C) under light shielded and light irradiation (black light, two FL20S BL-B 20W bulbs in parallel), with an ultraviolet light luminous flux of approximately 1 mW/cm$^2$ conditions.
After the above conditions were preserved for 24 hours, the SET-A was washed out from each sample with 10 mL of a buffer solution, which was included in the VIDAS staphylococcus enterotoxin (SET) test kit (bioMérieux, Inc.).
Furthermore, a plastic Petri dish, wherein a sample was not placed, was seeded with 0.4 mL of SET-A standard stock solution, and then 10 mL of buffer solution, which was included in the VIDAS staphylococcus enterotoxin (SET) test kit (bioMérieux, Inc.), was immediately added, and the result was used as the control.

**E.** Generation of the calibration curve

The concentration and the fluorescent light intensity of the calibration curve standard solution were measured by the ELISA method using a VIDAS staphylococcus enterotoxin (SET) test kit (bioMérieux, Inc.), and the calibration curve was generated based on those measurements.

**F.** Measurement of SET-A concentration and calculation of decomposition percentage

The fluorescent light intensity of the sample solution was measured by the ELISA method using a VIDAS staphylococcus enterotoxin (SET) test kit (bioMérieux, Inc.), the SET-A concentration was derived from the calibration curve generated in Step E, and the decomposition percentage was calculated by the following expression:

$$\text{Decomposition percentage (\%)} = \text{(Control measured value} - \text{Sample solution measured value)/Control measured value} \times 100$$

**(SUPPLY AIR AND EXHAUST AIR FLOWS)**

The total heat exchanging unit 100 is provided with three operation modes, i.e., a total heat exchange ventilation mode, a normal ventilation mode, and the air filter cleaning mode. The following describes the details of each of these operation modes.

**(1) TOTAL HEAT EXCHANGE VENTILATION MODE**

With the total heat exchanging unit 100, if total heat exchange ventilation is performed using the heat exchange element 12, then the damper 34 opens the opening 30. Furthermore, as discussed above, the opening 33 is blocked at this time. Furthermore, if the fans 10, 11 are operated in this state, the indoor air is suctioned via a duct from the indoor side suction port 4 into the indoor side suction chamber 27, passes through the exhaust air passageway 8, which extends from the opening 30 to the exhaust air communicating chamber 46, the fourth space 20, the air filters 12b, the heat exchange element 12, the first space 17, the opening 23, the exhaust air fan motor housing chamber 41, and the exhaust air fan housing chamber 22, and is then blown out from the outdoor side blow out port 7 and exhausted to the outdoor space via a duct; simultaneously, the outdoor air is suctioned via a duct from the outdoor side suction port 5 into the outdoor side suction chamber 26, passes through the supply air passageway 9, which extends from the supply air communicating chamber 45 to the third space 19, the air filters 12b, the heat exchange element 12, the second space 18, the opening 25, the supply air fan motor housing chamber 43, the opening 44, and the supply air fan housing chamber 24, and is then blown out from the indoor side blow out port 6 and supplied via a duct to the indoor space.

**(2) NORMAL VENTILATION MODE**

Normal ventilation, which does not perform heat exchange, is performed during intermediate seasons, such as spring and fall, when cooling and heating are not needed.

When normal ventilation is performed with the total heat exchanging unit 100, the damper 34 opens the opening 33. Furthermore, as discussed above, the opening 30 is blocked at this time. Furthermore, if the fans 10, 11 are operated in this state, the indoor air is suctioned via a duct from the indoor side suction port 4 into the indoor side suction chamber 27, passes through a bypass ventilation passageway, which extends from the opening 33 to the bypass chamber 31, the opening 32, the first space 17, the opening 23, exhaust air fan motor housing chamber 41, and the exhaust air fan housing chamber 22, and is then blown out from the outdoor side blow out port 7 and exhausted via a duct to the outdoor space; simultaneously, the outdoor air is suctioned via a duct from the outdoor side suction port 5 into the outdoor side suction chamber 26, passes through the supply air passageway 9, which extends from the supply air communicating chamber 45 to the third space 19, the air filters 12b, the heat exchange element 12, the second space 18, the opening 25, the supply air fan motor housing chamber 43, the opening 44, and the supply air fan housing chamber 24, and is then blown out from the indoor side blow out port 6 and supplied via a duct to the indoor space (the flow of the supply air is the same as for the case of total heat exchange ventilation).

**(FEATURES OF THE TOTAL HEAT EXCHANGING UNIT)**

**(1)**

With the total heat exchanging unit 100 according to the present embodiment, the heat exchange element 12 is a total heat exchanging type that comprises the moisture permeable spacer paper 122 and the partitioning paper 121. Furthermore, an air filter 12b is provided to the heat exchange element 12 not only on the supply airflow upstream side, but also on the exhaust airflow upstream side. In addition, both air filters 12b support titanium apatite. Furthermore, the catalytic function of the titanium apatite is activated by ultraviolet light lamps 15, which are provided nearby. Titanium apatite exhibits decomposition capacity with respect to, for example, bacteria, viruses and the like that is stronger than titanium dioxide of the anatase form. Consequently, the total heat exchanging unit 100 can prevent, more effectively than a conventional heat exchanging unit, contamination of the heat exchange element 12 due to, for example, dust, bacteria, viruses or the like suspended in the outdoor air.

**(2)**

According to the present embodiment, the ultraviolet light lamps 15 are provided to the total heat exchanging unit 100 in the vicinity of the air filters 12b. Consequently, the total heat exchanging unit 100 can also be disposed in outdoor and indoor where light does not reach.

**(3)**

With the total heat exchanging unit 100 according to the present embodiment, the catalytic function of the titanium apatite is activated by the ultraviolet light lamps 15. Accordingly, it is possible to hold down the manufacturing cost of the total heat exchanging unit 100.

**MODIFIED EXAMPLES**

**(A)**

With the total heat exchanging unit 100 according to the previous embodiment, the heat exchange element 12 is a total heat exchanging type that comprises the moisture permeable spacer paper 122 and partitioning paper 121, but the heat exchange element 12 may also be a sensible heat exchange type (a so-called heat pipe type heat exchange element) that comprises a moisture impermeable spacer plate and partition plate. Furthermore, with the sensible heat exchange type heat exchange element, only sensible heat is exchanged between the supply air and the exhaust air. In this case, a constitution may be adopted wherein an air filter 12b is provided only on the supply airflow upstream side. This is because, with the sensible heat exchange type heat exchange element, the supply air and the exhaust air do not mix, and therefore there is no risk that the dust, bacteria, viruses or the like contained in the exhaust air will mix into the supply air.

**(B)**

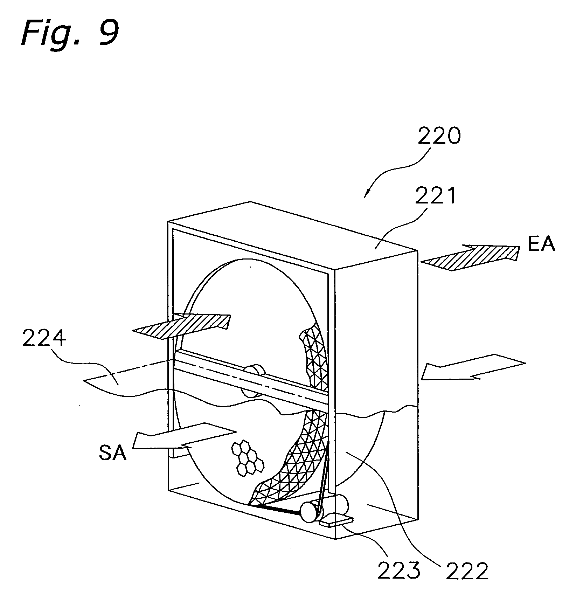

With the total heat exchanging unit 100 according to the previous embodiment, the heat exchange element is a fixed type, but may be a rotational type heat exchange element 220, as shown in FIG. 9. The rotational type heat exchange element 220 principally comprises a casing 221, a rotor 222, a gear motor 223, and a partition plate 224, as shown in FIG. 9. The casing 221 houses the rotor 222 and the gear motor 223. Furthermore, the rotor 222 is rotatably fixed to the casing 221. Furthermore, the gear motor 223 rotatably drives the rotor 222 via a belt. The rotor 222 has a honeycomb construction that comprises aluminum plates. Furthermore, a heat exchange element wherein the aluminum plates are coated with a moisture absorbent, for example, sodium chloride or a siliceous compound, is used as the total heat exchange heat exchange element, and one wherein the aluminum plates are not coated is used as the sensible heat exchange heat exchange element. The partition plate 224 partitions the rotor 222 in the vertical direction. In so doing, with the rotational type heat exchange element 220, the exhaust air passes through the upper half of the rotor 222, and the supply air passes through the lower half. In so doing, with the total heat exchanging type heat exchange element, the temperature and humidity of the exhaust air is higher than the outdoor air in, for example, the winter season, and the temperature and moisture content of the upper half of the rotor 222 itself rises due to the exhaust air. In this state, if the rotor 222 rotates and a portion that was in the area of the abovementioned upper half moves to the area of the lower half, then that portion contacts the supply air and throws off its temperature and humidity thereto.

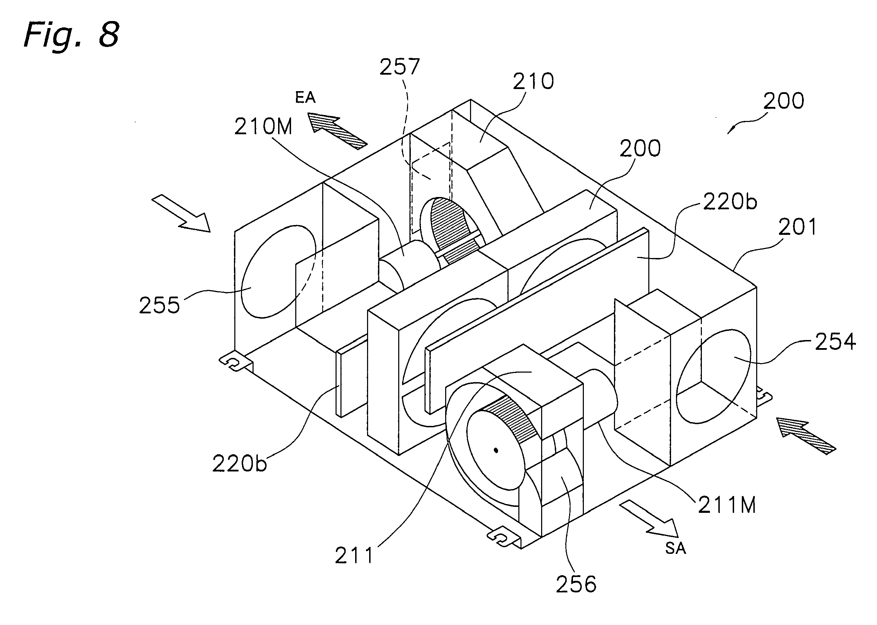

FIG. 8 shows a heat exchanging unit 200 wherein such rotational type heat exchange elements 220 are installed.

The heat exchanging unit 200 principally comprises a casing 201, the heat exchange elements 220, air filters 220b, an ultraviolet light lamp (not shown), and fans 210, 211, as shown in FIG. 8. In the heat exchanging unit 200, the heat exchange elements 220 are disposed in the vicinity of substantially the center of the casing 201 in the longitudinal direction so that the rotational axes of the rotors 222 coincide with the longitudinal direction. In addition, in the heat exchanging unit 200, two rotors 222 are disposed lined up in a direction orthogonal to the longitudinal direction. Furthermore, the air filters 220b are disposed on the exhaust airflow upstream side and on the supply airflow upstream side of the rotor 222. Furthermore, the air filter 220b that is disposed on the exhaust airflow upstream side of the rotors 222 covers the upper half of the rotors 222. In addition, the air filter 220b that is disposed on the supply airflow upstream side of the rotors 222 covers the lower half of the rotors 222. In addition, the air filters 220b are the same as the air filters 12b according to the previous embodiment. In addition, titanium apatite is disposed in the vicinity of the air filters 220b, and the ultraviolet light lamp (not shown) activates its catalytic function. The exhaust air fan 210 is provided on the outdoor side of the heat exchanging unit 200 and is driven by an exhaust air fan motor 210M. Moreover, the supply air fan 211 is provided on the indoor side of the heat exchanging unit 200 and is driven by a supply air fan motor 211 M.

When the fans 210, 211 of the heat exchanging unit 200 operate, the indoor air is sucked into an indoor side suction port 254 via a duct, passes through an exhaust air passageway that extends from the air filters 220b to the heat exchange element 220 and the exhaust air fan 210, is then blown out from an outdoor side blow out port 257 and exhausted to the outdoor space via a duct; simultaneously, outdoor air is sucked into an outdoor side suction port 255 via a duct, passes through a supply air passageway that extends from the air filters 220b to the heat exchange element 220 and the supply air fan 211, and is then blown out from an indoor side blow out port 256 and supplied to the indoor space via a duct.

**(C)**

The total heat exchanging unit 100 according to the previous embodiment employs the ultraviolet light lamps 15 in order to activate the catalytic function of the titanium apatite, but an LED may be used instead. Doing so can contribute to energy conservation and a reduction in the global environmental load (because mercury is not used in the LED), and can also hold down manufacturing costs.

**(D)**

The total heat exchanging unit 100 according to the previous embodiment uses the ultraviolet light lamps 15 in order to activate the catalytic function of the titanium apatite; however, if the total heat exchanging unit 100 is installed in outdoor or indoor where light reaches, then at least one of the casing 1 and the cover body 3 may be made transparent, and the titanium apatite may be activated by at least one of outdoor light and indoor light.

**(E)**

The total heat exchanging unit 100 according to the previous embodiment uses the ultraviolet light lamps 15 in order to activate the catalytic function of the titanium apatite, but a streamer discharger, a glow discharger, a barrier discharger, or the like may be used instead. By disposing such a discharger in the total heat exchanging unit 100, it is possible to supply, for example, ozone and high energy radicals that have a bactericidal effect on the titanium apatite. Accordingly, the total heat exchanging unit 100 increases the bactericidal effect and can also accelerate the photocatalytic reaction speed of the titanium apatite. As a result, the heat exchanging unit 100 can prevent, more effectively than a conventional heat exchanging unit, the contamination of the heat exchange element 12 due to, for example, dust, bacteria, or viruses that are suspended in the outdoor air.

Furthermore, if the total heat exchanging unit 100 is provided with a plasma discharger, then an air filter cleaning mode may be made available in addition to the total heat exchange ventilation mode and the normal ventilation mode. In the air filter cleaning mode, the rotational speed of the fans 10, 11 is controlled so that it transitions to a state wherein the amount of ventilation is suppressed as much as possible. Furthermore, the plasma discharger may be constituted so that it is energized only in this mode, or so that it is continuously energized. In so doing, a high concentration of radicals is supplied to the air filters 12b. Accordingly, with the total heat exchanging unit 100, it is possible to selectively and efficiently purify the air filters 12b.

**(F)**

With the total heat exchanging unit 100 according to the previous embodiment, titanium apatite is supported in a form wherein it is embedded in the enveloping layer of the polypropylene fibers, but it may be coated thereon.

**(G)**

With the total heat exchanging unit 100 according to the previous embodiment, polypropylene fibers are used as the support body of the titanium apatite, but the support body of the titanium apatite is not limited thereto. For example, it is possible to use various kinds of plastic fibers and natural fibers.

**(H)**

With the total heat exchanging unit 100 according to the previous embodiment, titanium apatite is supported in a form wherein it is embedded in the enveloping layer of the polypropylene fibers, but it may be coated on one or both sides of each air filter 12b.

**INDUSTRIAL APPLICABILITY**

A heat exchanging unit according to the present invention can prevent, more effectively than a conventional heat exchanging unit, the contamination of a heat exchange element due to, for example, dust, bacteria, viruses or the like suspended in the outdoor air, and can also be adapted to applications such as an air conditioner or an air cleaner.

**Claims**

1. A heat exchanging unit (100, 200), comprising:

   an indoor air exhaust passageway (8) that exhausts indoor air to an outdoor space as exhaust air (EA);
   an outdoor air supply passageway (9) that supplies outdoor air to an indoor space as supply air (SA);
   a heat exchange element (12, 220) that communicates with said indoor air exhaust passageway (8) and said outdoor air supply passageway (9), and exchanges at least the sensible heat of said sensible heat and the latent heat between said exhaust air (EA) and said supply air (SA); and
   a first air cleaning member (12b, 220b), which is provided on the supply airflow upstream side of said heat exchange element (12, 220), that supports an apatite that has a photocatalytic function.

2. A heat exchanging unit (100, 200) as recited in Claim 1, further comprising:

   a second air cleaning member (12b, 220b), which is provided on the exhaust airflow upstream side of said heat exchange element (12, 220), that supports said apatite that has said photocatalytic function.

3. A heat exchanging unit (100, 200) as recited in Claim 1 or Claim 2, further comprising:

   a photocatalytic function activating part (15) that activates said photocatalytic function of said apatite.

4. A heat exchanging unit (100, 200) as recited in Claim 3, wherein
said photocatalytic function activating part (15) irradiates said apatite that has said photocatalytic function with light in a predetermined wavelength range.

5. A heat exchanging unit (100, 200) as recited in Claim 3, wherein
said photocatalytic function activating part is provided on the airflow upstream side of said air cleaning member (12b, 220b), and supplies activators to the apatite that has said photocatalytic function.

6. A heat exchanging unit (100, 200) as recited in Claim 5, further comprising:

an air distribution part (10, 11, 210, 211) that distributes said air; and
an operation switching unit (EB) that switches the operation state of said air distribution part (10, 11, 210, 211).

Fig. 1

EP 1 736 712 A1

# Fig. 2

*Fig. 3*

# *Fig. 4*

## Fig. 5

(16E)16C

29

## Fig. 6

42(44)

16B(16D)

# *Fig. 7*

Fig. 8

Fig. 9

<table>
<tr><td colspan="2" align="center">**INTERNATIONAL SEARCH REPORT**</td><td colspan="2">International application No.<br>PCT/JP2005/005727</td></tr>
</table>

| | |
|---|---|
| A. CLASSIFICATION OF SUBJECT MATTER<br>Int.Cl⁷ F24F3/16, A61L9/00, 9/01, 9/20, B01D53/86, B01J35/02, F24F3/147, 7/08 | |

According to International Patent Classification (IPC) or to both national classification and IPC

**B. FIELDS SEARCHED**

Minimum documentation searched (classification system followed by classification symbols)
Int.Cl⁷ F24F3/16, A61L9/00, 9/01, 9/20, B01D53/86, B01J35/02, F24F3/147, 7/08

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho    1971-2005   Toroku Jitsuyo Shinan Koho   1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 10-311581 A (Matsushita Seiko Co., Ltd.),<br>24 November, 1998 (24.11.98),<br>Full text; all drawings<br>(Family: none) | 1-6 |
| Y | JP 2000-327315 A (Fujitsu Ltd.),<br>28 November, 2000 (28.11.00),<br>Full text<br>(Family: none) | 1-6 |
| Y | Nihon Keizai Shimbun, 17 July, 2003 (17.07.03),<br>Morning Paper, page 15 | 1-6 |
| Y | JP 2001-121031 A (Yasuro ITO),<br>08 May, 2001 (08.05.01),<br>Par. No. [0029]<br>(Family: none) | 5,6 |

☐ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>07 June, 2005 (07.06.05) | Date of mailing of the international search report<br>28 June, 2005 (28.06.05) |
|---|---|
| Name and mailing address of the ISA/<br>Japanese Patent Office | Authorized officer |
| Facsimile No. | Telephone No. |

Form PCT/ISA/210 (second sheet) (January 2004)

**EP 1 736 712 A1**

## REFERENCES CITED IN THE DESCRIPTION

**Patent documents cited in the description**

- JP H10311581 A